# EUROPEAN PATENT SPECIFICATION

(11) **EP 0 570 714 B1**
(45) Date of publication and mention of the grant of the patent: **01.09.1999**
(21) Application number: 93106529.6
(22) Date of filing: 22.04.1993
(51) Int. Cl.: A61K 31/16

(54) **Use of aminoalcohol-N-acylderivatives for the manufacture of a medicament for the treatment of neurogenic endoneural oedema of the peripheral nerve**
Verwendung von N-Acylderivaten von Aminoalkoholen zur Herstellung eines Arzneimittels zur Behandlung von neurogenen endoneuralen Ödemen der peripheren Nerven
Utilisation de derivés N-acyle d'aminoalcools pour la fabrication d'un médicament pour le traitement d'oedèmes endoneurales neurogènes au niveau des nerfs périphériques

(30) Priority: 24.04.1992 IT MI920979
(43) Date of publication of application: 24.11.1993
(73) Proprietor: LIFEGROUP S.p.A., I-35043 Monselice (Padova) (IT)
(72) Inventor: Della Valle, Francesco, I-35122 Padova (IT); Lorenzi, Silvana, I-35100 Padova (IT); Della Valle, Federica, I-35123 Padova (IT)
(74) Representative: Gervasi, Gemma, Dr.

(56) References cited:
- F.PERLIK ET AL. 'future trends in inflammation' 1974 , PICCIN MEDICAL BOOKS , VERONA,LONDON,PARIS * page 301 * * page 305, line 1-2 *
- J.ALLERGY CLIN.IMMUNOL. vol. 86, no. 4(2) , 1990 pages 677 - 683 JOANN M. MICAN ET AL. 'Arthritis and mast cell activation'
- NEUROSCIENCE LETTERS vol. 69 , 1986 pages 296 - 301 INGER NENNESMO ET AL. 'Mast cells in nerve and neuromas of mice'
- LABORATORY INVESTIGATION vol. 48, no. 3 , 1983 pages 332 - 338 HENRY C. POWELL ET AL. 'Early changes in experimental allergic neuritis'
- INT.REV.CITOL. vol. 24 , 1968 pages 27 - 70 YNGVE OLSSON 'Mast cells in the nervous system'
- JACS, vol. 79, no. 19, 1957, page 5577-5578

## Description

### FIELD OF THE INVENTION

The present invention relates to the use of aminoalcohols N-acyl derivatives for preparing pharmaceutical compositions for the prevention or the therapeutic treatment of human and animal pathologies involving a neurogenic endoneural oedema induced by mast cells degranulation as a consequence of a peripheral nerve noxa.

### PRIOR ART DISCLOSURE

It is known that noxae of different origin in the peripheral nervous system determine significant alterations both in the neural components and in the microenvironment of the injured nerve.

From a strictly anatomical point of view the peripheral nerve is a complex structure of neural components, namely Schwann cells and axons, and non-neural components, that is connective matrixes, extracellular fluids and blood vessels. The extracellular fluids, together with the connective matrixes form the microenvironment, through which the exchanges of the exogenous nutritious substances and endogenous substances produced by the same neural cells (mainly the Schwann cells, because analogous exchanges between the axons and the blood is accomplished through the axoplasmatic flow) occur between the blood and these neural cells.

These two components, e.g.the microenvironment and the neural portion, together form a nervous fasciculus which structurally results to be separated by the perineurium from the surrounding tissues, so that a tubular structure originates, being physically delimited by the perineural barrier functionally comparable with the hematoencephalic barrier. The purpose of this barrier is to regulate the fine homeostasis between the inside (endoneurium) and the outside (epineurium); the perineurium has therefore the function of a quali-quantitative homeostatic regulation of the microenvironment fluids with respect to the external ones.

Another structurally and fundamentally important element for the homeostatic equilibrium maintenance of the microenvironment extracellular fluids consists in "vasa nervorum", the blood vessels through which the blood arrives at the nerves.

The endoneural extracellular fluids are in fact in equilibrium with blood serum, which arrives through these vessels.

Blood vessels are in turn innervated by free nerve endings, the so called "vasa nervorum". In other words they are intramural terminations innervating the vasa nervorum, having essentially a peptidergic origin. The main peptides contained in these terminationes are substance P, bradykinin, VIP and NPY. These terminations also contain conventional neurotransmitters such as serotonin and dopamine ( Appenzeller O. et al. , Brain Res. 1984, 304(2) ,383-386).

Under many lesive conditions of the peripheral nerve, having independently traumatic, toxic or dysmetabolic causes, a hyperactivation situation is observed for these nervous endings which release high amounts of the aforementioned neuropeptides, locally active in the area determined in the epineural barriers. The etiopathogenetic meaning of this nervous hyperstimulation becomes clear, if one recognizes that a narrow microanatomic relationship exists between the nervous peptidergic endings and particular immunocomponent cells, like the mast cells (Heine, H. et al., Z. Mikroskop. Anat. Forsch. 1975, 89,934-937).

In this regard it is important to remember that mast cells are widely present in the peripheral nerve both in the epineurium where they are located along the blood vessels, and in the perineurium where they are associated, too, with the capillary vessels. Mast cells are also present in the endoneurium, more in the distal portion of the nerve than in the proximal one (Olsson, Y. Int. Rev. Citol. 1968, 24,27-70).

It is furthermore important to point out that the most recent research has demonstrated a dramatic increase of RNA messenger for the NGF (nervous growth factor), as well as the NGF protein synthesis occurring in neural and non neural cells (Schwann cells and fibroblast type cells) associated with the nerve involved in the lesion (Lindholm, D. et al Nature 1987, 330, 658-659); the above mentioned phenomena are regulated by the synthesis of a cytokine: interleukin-1, produced by the activated macrophages (Wehling, P. et al Neuro-orthopedics 1989,7, 55-59). This marked increase of NGF in the lesion site determines in its turn a considerable increase of the number of local mast cells number acting as "gate keepers", and therefore as amplifiers of the morphofunctional phenomena as a consequence of a lesive noxa.( Aloe.L., Levi Montalcini R., Brain Res. 1977, 133, 358-366; Aloe L et al. Olin. Exp. Rheumatol. 1992, 10, 203-204).

The peptidergic nervous fiber-mast cell functional unit is thus involved in many paraphysiologic events such as pain, neurogenic inflammation and the local regulation of the blood flow (Foreman, J.C. Allergy 1987, 42, 1-11). It is in fact important to remember that the substance P release induced by nervous stimulation causes a mast cell hyperactivation condition able to determine degranulation of the preformed mast cells stores of mediators such as histamine, serotonin, tumor necrosis factor (TNF), involved in the induction, amplification of the inflammatory processes and formation of local oedema.

Under many conditions of the peripheral nerve injury caused or associated with inflammatory processes alterations of the perineurium are observed, but above all very significant changes are noted both in the composition and in the amounts of endoneural fluids with impressive oedema conditions, which, although generally representing a consequence of the nervous stimulation induced by the injury and therefore not being its primary cause, actually determine a significant worsening of the pathologic situation.

Following to the neurogenic oedema onset, significant alterations of the vascular permeability, both of the endoneural and of the perineural microvessels take place, thus compromising the protective function of the perineural barrier.

The oedema can occur at the level of any nervous compartment; in other words following to a noxa a fluids accumulation may be observed at the perineural, epineural level or again at the endoneural one.

Besides the anatomic localization, it is important to distinguish between the oedema characterized by an alteration of the vasal permeability to proteins with their consequent extravasation, and the oedema not having this protein leakage.

Under many conditions of peripheral nerve injury, a consistent pattern of endoneural neurogenic oedema take place together with a remarkable protein extravasation as a consequence of the nervous hyperstimulation.

During the neurogenic endoneural oedema formation, the first crucial event consists of the perineural barrier nerve / blood breaking , determining a precocious protein leakage, wherein, as above said, mast cells activation and the consequent release of the endogenous neurotransmitters play a key role. Mast cell degranulation, following peripheral nerve traumas with consequent mediators release, such as histamine, can increase the vasal permeability which in the first place is the main cause of the oedema formation ( Olsson Y. Int Rev. Citol. 1968,24,27; Nennesmo I. et al . Nuerosci, Lett.1986,69, 296).

Actually the same mediators, successively in association with inflammation specific mediators, after causing a rapid increase of the endoneural pressure for the oedema formation, slow down the vascular permeability, by further increasing the endoneural pressure, thus determining secondary degenerative type injuries (Mellick, R.S. et al. , J.Neurol. Neurosurg. Psychiatry 1967, 30, 458.

The neurogenic endoneural oedema has therefore important implications from a pathogenic point of view under many peripheral, somatic and autonomic neuropathological conditions.

In fact, besides slowing down the endoneural microcirculation, therefore blocking the nutritious substances supplied by a mechanical type action to the neural components, which, as a consequence of that, lead to death, the oedema condition favours, in presence of a high protein concentration deriving from the protein leakage increase, a remarkable production of collagen and of a cultural medium, being suitable for the proliferation of fibroblasts, mast cells and Schwann cells, which are able to induce a fibrosis aiming to compromise the nerve functionality.

Therefore, the oedema formation at the level of the microenvironment (matrix and extracellular fluids) is associated to other pathologically important alterations, as for example fibroblasts and mast cells proliferation, alterations which may assume a primary pathogenic role.

It is finally important to point out that, following the neurogenic oedema formation, a vicious circle starts, where mast cells play a primary role in the induction (first phase of the oedema induced by mast cell degranulation) and in the worsening of the oedema state (second phase of the oedema associated with fibrosis and mast cells proliferation).

In other words following to a peripheral nerve lesion an immediate first type cellular reaction occurs in the lesion site, identifiable as a degranulation process of the endoneural mast cells, followed by a release of mediators which results in an oedema formation. As a consequence of the oedema condition, a process of cellular proliferation starts, where an important component is again represented by mast cells. In this second phase also distal alterations, with respect to the primary site of lesion, known as Wallerian degeneration, appear at the level of the nerve. Also in the case of the Wallerian degeneration the increase in mast cells proliferation is considerably higher in the endoneural compartment than in the peri- and epineural one, occurring in the distal portion of the nerve. (Olsson Y. et al., Acta Neuropath. 1969, 13, 111).

The oedema formation is in any case associated with an impressive alteration of the perineurium permeability at the lesion site (Olsson Y et al., Rev Adv, Neuropathol. 1979, 2,1).

This modification starts very quickly during the first hour from the injury and lasts for some weeks, but it seems to involve only the proximal portion of the lesion and not the distal one and it does not seem to be connected with a protein leakage. Furthermore the perineurium oedema does not seem to be sensitive to the inflammation mediators. Therefore from a pathologic point of view the neurogenic endoneural oedema results to be of primary importance in the peripheral nerve pathologies, due to these above mentioned features.

It appears evident from the above that from a therapeutical point of view it would be very important to have compounds able to control mast cell hyperactivation and degranulation as a consequence of the peripheral nerve noxae of various origin, determining the onset of the neurogenic endoneural oedema.

The involvement of mast cell degranulation in the pathogenesis of rheumatic diseases, in particular of rheumatoid arthritis, and in the outset of the inflammatory process in experimental allergic neuritis, accompanied by endoneural oedema formation, is known in the state of the art (J.M. Mican et al., J.Allergy Clin. Immunol., vol.86, 4: 677-683, 1990; H.C. Powell et al., Laboratory Investigation, vol. 48, 3:332-338, 1983).

Quite recently the Applicant has found compounds active in modulating mast cells degranulation processes, acting with a local antagonist autacoid type mechanism of action, and which can be advantageously used in the treatment of autoimmune pathologies, as described in the European Patent Application 92121862.4.

It is in fact known that, in autoimmune diseases, pathologic autoaggressive phoenomena occur, revealing themselves through processes of localized tissue damage where specific immunocompetent cells, and among them mast cells, play a central aetiopathogenetic role.

Mast cells are in fact a cellular population residing in tissues which, self activating in situ, proceed to predetermine the biologic sequence of the inflammation, releasing several neurotransmitters, often characterized by a considerable cytotoxicity, and are responsible for the localized tissue damage.

Anyway it is known that mast cells activity is regulated in agonist manner by neuro and immuno-mediated activating systems, antagonized by inhibitory systems, revealing themselves through general circuits, as for example corticosteroid hormones.

The Applicant has found that the compounds derived from the N-acylation of aminoalcohols such as mono and diethanolamine, can act on mast cells as antagonist local systems, through an autacoid type mechanism, and as a consequence of that, they can be advantageously used in the therapy of autoimmune pathologies.

Also N-(2-hydroxyethyl)hexadecanamide, or N-palmitoylethanolamide (N-PEA), belongs to this class of compounds. The activity of this compound was casually discovered around the Fifties, in consequence of the identification of a generic cytoprotective activity of the lipidic excipient, in which this compound was present, of an antirheumatic drug.

The pharmacologic profile of this compound was then studied in experimental patterns by following damages caused by different agents; the interesting ability in increasing the resistance of the animals to various bacterial toxins was deemed the most suitable for the successive pharmaceutical development of this compound. In fact it is on this base that a pharmaceutical product in the form of tablets was launched in Czechoslovakia, whose therapeutical indication was the prevention of infections of the respiratory tract.

F.A. Kuehl Jr. et al. (J.Am.Chem.Soc., vol. 79, 19:5577-5578, 1957) described the generic anti-inflammatory activity of N-PEA and attributed said activity to the alcoholic moiety of the molecule (i.e. ethanolamine).

F. Perlik et al. (Future Trends in Inflammation, Piccin Medical Books, pages 301-306, Verona 1974) investigated the effect of N-PEA on inflammatory processes, such as rheumatoid arthritis; neither a teaching concerning the activity of said compound on mast cells, not the involvement of mast cells in the above pathologies was reported.

With respect to what is already known, the Applicant has in fact found that both the activity of N-PEA, and that of the compounds belonging to the wide class of amino alcohols N-acyl derivatives described in the European Patent Application above cited, is not only directed to a generic and moderate cytoprotective activity, as suggested by the prior art for N-PEA, but on the contrary said class exhibit a specific and important role in the inhibitory modulation of mast cell degranulation and therefore in inhibiting the autoaggressive effects of the autoimmune pathologies. This is the consequence of the inhibition of the uncontrolled release of preformed mast cell granules,containing numerous proinflammatory mediators and in particular preformed granules containing the Tumor Necrosis Factor, a highly cytotoxic cytokine involved in the autoaggressive autoimmune process (Toms R. et al., J.Neuroimmunology,1990,30, p.169-177; Kruger P.G. et al., Acta Neurol. Scand., 1990, 81, p.331-336).

What is described in the above mentioned European patent application does not imply an involvement of these compounds on the neurogenic endoneural oedema under peripheral nerve damage conditions, since the endoneural compartment results to be effectively protected by the barrier formed by the perineurium, whose function was previously described. Therefore it does not result as an obvious consequence the ability of the same compounds in blocking endoneural mast cells degranulation, induced by a nervous hyperstimulation on lesive base of the peripheral nerve. In any case it is not evident on the basis of what is already known or previously discovered by the same Applicant that these compounds can act as local antagonist autacoids, also at the level of endoneural mast cells of the peripheral nerve.

And in any case this action is more and more important if we consider that, following to a primary effect in inhibiting mast cells degranulation, these compounds are not only able to prevent the endoneural oedema formation, but also to prevent mast cells proliferation, which is the determining event of starting the oedema vicious circle being the responsible of the chronicity and the seriousness of the pathology.

### SUMMARY OF THE INVENTION

The Applicant has in fact found that the degranulation and the consequent mast cells proliferation occurring following traumatic, toxic or dysmetabolic noxae of the peripheral nerve, can be effectively antagonized by administering aminoalcohols N-acyl derivatives, as these compounds are able to control the neurogenic endoneural oedema, associated with pathologic states of the peripheral nerve.

The present invention therefore relates to the use of said compounds in the preparation of pharmaceutical compositions for the prevention or the treatment of pathologies of the peripheral nerve, having traumatic, toxic or dysmetabolic origin, characterized or associated with neurogenic endoneural oedema.

### DETAILED DESCRIPTION OF THE INVENTION

The characteristics and advantages of the aminoalcohols N-acyl-derivatives of the present invention, acting as local autacoids and therefore utilizable in the treatment of pathologies of the peripheral nerve system, characterized by a neurogenic endoneural oedema, will be better understood in the light of the instant detailed description.

The Applicant has surprisingly found that the activity of N-PEA is not limited to a generic cytoprotective activity, as suggested by the prior art, but that this compound, being able to modulate also mast cells degranulation in this specific and protected neurogenic endoneural compartment, plays a specific and important role in controlling the neurogenic and endoneural oedema in this compartment of the peripheral nerve.

For this reason this compound is utilizable in the preparation of pharmaceutical compositions suitable to be administered in man and animal for the treatment of pathologies of the peripheral nerve as a consequence of noxae of different (e.g traumatic, toxic or dysmetabolic) origin.

More generally, this pharmacological activity being related to mast cells degranulation modulation mechanism , which the whole class of the aminoalcohols N-acyl derivatives resulted active to, the same activity can be claimed not only for N-PEA, but for the entire class of the compounds according to the present invention. In order to define better the present invention, the compounds belonging to the above mentioned class of the N-acyl derivatives are characterized by the following formula: 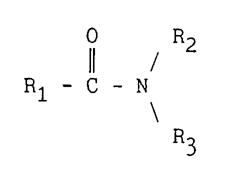 wherein R₂ is an alcoholic residue selected from a C₁-C₂₀ linear or branched hydroxyalkyl, optionally substituted in the alkyl chain with at least one aryl group, and a hydroxyaryl, optionally substituted on the aromatic ring with at least one linear or branched alkyl radical of from 1 to 20 carbon atoms; wherein R₃ is H or is = R₂; and wherein 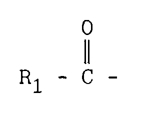 is an acyl radical of a monocarboxylic acid.

Preferred alcoholic residue R₃ and/or R₂ according to the present invention, for a merely illustrative but not limitative purpose, are those of monoethanolamine, diethanolamine, 2-hydroxypropylamine, di-(2-hydroxypropyl)-amine, which bring to the compounds having the following formulas:

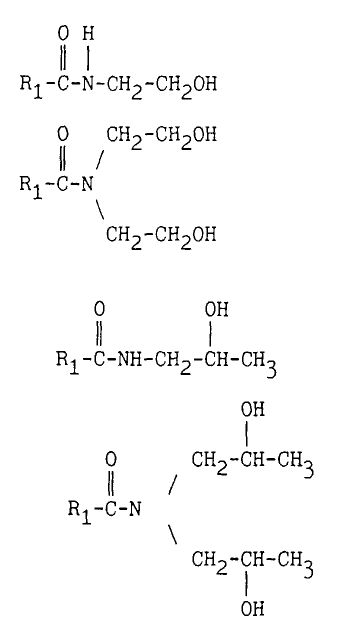

The N-acylderivatives of 2-hydroxy-propylamine and of di-(2-hydroxy-propyl)-amine may be an optical isomer or a raceme. As regards the acyl portion of the molecule of the N-acyl derivatives according to the present invention , namely the R₁-CO group, it is preferably the acyl radical of a monocarboxylic acids. Among these, the monocarboxylic saturated or unsaturated acids, optionally substituted with a hydroxy or aminic group on the aliphatic chain, and the monocarboxylic acids containing aromatic, heteroaromatic and/or heterocyclic groups are to be considered.

For illustrative purposes, in the group of the monocarboxylic aliphatic acids suitable to acylate the nitrogen of the above mentioned aminoalcohols are to be considered all the acids of biological importance as for example palmitic, oleic, stearic, lauric, myristic and their hydroxy and amino homologues.

Among the acids containing aromatic, heteroaromatic and/ or heterocyclic groups the following can be used : salicylic, acetylsalicylic, sulfosalicylic, benzoic, trimethoxybenzoic, α-lipoic (= thioctic acid), retinoic, thenoic, phenylanthranilic, hydroxyphenylacetic and all the biologically acceptable acids.

The following examples describe the subject of the present invention and the best way to carry out the invention, but in no way they limit it.

### Example 1- N-palmitoylethanolamide (N-PEA) synthesis

Following Roe E.T. et al. indications (J. Am Chem. Soc., 1952, 74, 3442-3443), N-palmitoylethanolamide synthesis was accomplished by reacting under reflux ethanolamine with palmitic acid. Particularly 1 mole of palmitic acid was reacted in the presence of 1.5 moles of ethanolamine in ethyl ether for 5-6 hours under nitrogen atmosphere.

The reaction product was then extracted from the reaction mixture and crystallized by using 95 % ethanol at a temperature of 0 °C. The melting point found for N-PEA was around 94-95 °C.

The physical chemical characteristics of the N-PEA product synthesized according to the present example were the following :

| | |
|---|---|
| - physical state | crystalline powder |
| - raw formula | C₁₈H₃₇NO₂ |
| - molecular weight | 299.48 |
| - elemental analysis | C72.19%; H12.45%; N 4.68%; O10.69% |
| - solubility in organic solvent | Hot MetOH, CHCl₃, DMSO |
| - solubility in water | insoluble |
| - melting point | 94-95 °C |
| - TLC in chloroform/methanol | (9:1) Rf = 0.75 |

N-Palmitoyldiethanolamide and the other compounds of the invention were prepared by a similar process, as described in the above mentioned European Patent Application 92121862.4.

### i) Biological activity against mast cell degranulation of the peripheral nerve.

In order to verify the pharmacological activity of N-acyl derivatives of aminoalcohols, and in particular of N-PEA, under conditions of traumatic noxa due to crushing of the sciatic nerve, and therefore able to induce mast cells degranulation, the following "in vivo" biologic test was carried out.

### Materials and Methods

Male Spraugue Dawley Rats weighing about 200 -250 g were divided into two groups and subjected for 15 days to two different treatments: the first group, being the control group, was intraperitoneally treated with a solution of liposomes of soy lecithin, whereas the second group was intraperitoneally treated with 20 mg/kg N-PEA vehiculated on soy lecithin liposomes. The choice of this specific vehicle depended on the poor solubility of N-PEA in aqueous vehicles.

At the 16^{th} day the animals were subjected to a bland general anesthesia with ethyl ether, thereby proceeding to the operation of the nerve lesion as hereinbelow described. The left sciatic nerve was exposed just in the spot of its entrance to the haunch, by operating a dissection of the muscular bands. The thus exposed sciatic nerve was then repeatedly compressed as closest as possible to its origin, by using a crushing forceps. The wound was temporarily sewn up and the animals were left to recover.

Three hours after the sciatic nerve compression lesion the animals were sacrificed in order to take the sciatic nerve samples and to fix it for the histological analysis with a mixture of acetic acid and formalin. The tissues were then cut into fine sections, colored with a masts cell specific dye, 5% toluidine blue in a citrate buffer at pH 4.5, as described by Enerback L., et al. (Zeit. Zellforsch. 1965, 66, 596). The thus prepared tissues were then examined at the optical microscope in order to count mast cells.

### Results

With the purpose of evaluating the pharmacologic effects of NPEA, a comparative analysis of the number of degranulated mast cells of the two rats groups was conducted.

For each animal, a total nummber of 100 cells in sections in the proximity of the sciatic nerve lesion were examined.

The results are hereinbelow reported in tables 1 and 2:

| Groups | Number of degranulated cells on a total of 100 examined cells |
|---|---|
| CONTROL: | |
| rat No.1 | 43 |
| rat No.2 | 74 |
| rat No.3 | 65 |
| rat No.4 | 68 |
| rat No.5 | 57 |
| average | 61.4 |

| Groups | Number of degranulated cells on a total of 100 examined cells |
|---|---|
| N-PEA 20 mg/kg | |
| rat No.1 | 35 |
| rat No.2 | 44 |
| rat No.3 | 37 |
| rat No.4 | 25 |
| rat No.5 | 42 |
| average | 36.6 |

As it results from the foregoing, N-PEA is also able to modulate mast cell degranulation processes activated by a traumatic compressive type noxa. In this way it is able to exert a pharmacological effect on neurogenic oedema induced by mast cells activation process.

The pharmacologic activity of N-PEA, common to the entire class of the aminoalcohols N-acylderivatives whose N-PEA represents the first example, plays a significant role in all the pathologic states of the peripheral nervous system wherein, in consequence of a peripheral nerve noxa both of traumatic type and of toxic or dysmetabolic origin, a sufferance of the nerve occurs, associated with a neurogenic endoneural oedema. In practice this is a category of pathologies universally known as peripheral neuropathies, according to a definition given by the World Health Organization (WHO) in WHO-Study group held in Geneva in October 1979, and contained in a Specific Technical Report (Peripheral Neuropathies-Technical Report Series No. 654-WHO 1980). Although the causal agents of peripheral neuropathies are various (varying from chemical agents such as solvents, pesticides, viral or bacterial agents [herpes zoster, HIV, diphtheric toxin], to metabolic causes such as diabetes and uremia and to traumatic-compressive causes such as those occurring in intravertebral disc protrusion), as to their classification the World Health Organization divides peripheral neuropathies into three main categories : neuropathies having traumatic, toxic and dysmetabolic origin. Generally the compounds according to the present invention are parenterally (intravenously, intramuscularly and subcutaneously) or orally administered. In any case under anatomically localized neuropathic conditions, such as localized traumatic lesions, topic (dermic, intra- and trans-dermic) administrations are not to be excluded.

The therapeutically effective doses vary depending on the administration route and on the application modalities, as well on the pathology severity; other factors are also to be considered connected with the patients' age, weight and health general conditions. A therapeutically acceptable range may be any way comprised between 0.1 and 50 mg/kg and preferably between 1 and 20 mg/kg. Relevant undesired effects of the compounds according to the present invention being unknown, further to the therapeutic dose also a therapeutic regimen has to be established on the base of medical criteria, taking into account the acuteness or chronicity characteristics of the pathology.

Typically a therapeutic regimen may be of from 1 to 2 daily administrations of the drug for 3-4 weeks, even in therapeutic cycles repeated for at least three months. Anyway, under particular acute neuropathic states, short duration regimen are not to be excluded, being characterized by 3-4 daily administrations for 1 or 2 weeks.

Furthermore, as these pathologies are characterized by new acute phases, the aminoalcohol N-acylderivatives according to the present invention can be advantageously used for a preventive action, as well as for a therapeutic one.

As preventive agents in chronic pathologies characterized by recurrent acute episodes, these compounds can be administered as dietetic integrating components, and the daily dosage foreseen for this specific use of the compounds according to the present invention preferably range from 0.1 to 1 mg/kg, both for human beings and for animals.

The compounds according to the present invention can be formulated in pharmaceutical compositions comprising all those substances suitable for the above mentiones administrations and the excipients can be those pharmaceutically and therapeutically suitable for the same applications as well those of a more recent conception able to improve the delivery of these active principles to the site of action.

The preferred formulations for the topical (dermic, intra- and trans-dermic) administration are buffered solutions, gels, medicated plasters, whereas for the systemic oral administration all the formulations are suitable in the form of dry powders such as granulates, tablets, dragees, soft or hard jelly perles, containing the compounds dissolved or dispersed in lipidic excipients; in the liquid form as suspensions, aqueous solutions or emulsions wherein these compounds are present even in the form of liposomes, optionally mixed with other lipids; in semisolid form obtained with polysaccharides aqueous suspensions, solutions or emulsions containing the compounds according to the present invention in the form of liposomes optionally mixed with other biologically acceptable lipids.

The dietetic integrating components are preferably in the form of dragees, tablets or oily perles.

For the rectal administration, dispersions of the compounds according to the present invention can be utilized in the form of dispersions in an acceptable phospholipidic excipient.

As to the parenteral administration, the preferred formulations are aqueous buffered solutions or emulsions, containing the compounds of the present invention optionally in the form of simple or mixed liposomes with other biologically acceptable lipids for the parenteral use, or oily solutions; these parenteral formulations can also consist of a lyophilized product readily dispersable in the solvent at the moment of the administration.

With the aim to rendering evident the possible industrial use of the compounds according to the present invention we report hereinbelow some examples of preferred pharmaceutical compositions.

These composition are only provided for illustrative, but they are in no way to be considered as limitative of the present invention.

| Example 1 Tablets : | |
|---|---|
| Every tablet contains : | |
| N-palmitoylethanolamide | 300 mg |
| lactose | 35 mg |
| maize starch | 90 mg |
| polyethylene glycol | 5 mg |
| carboxymethyl cellulose | 15 mg |
| magnesium stearate | 5 mg |
| Yellow Fe oxyde (E172) | 0.2 mg |

| Example 2 Soft Jelly Perles : | |
|---|---|
| Every perle contains : | |
| N-palmitoylethanolamide | 100 mg |
| soy lecithin | 40 mg |
| peanut oil | 100 mg |
| jelly | 52 mg |
| glycerol | 16 mg |
| erythrosin (E127) | 0.1 mg |

| Example 3 Chewable Jelly Drops : | |
|---|---|
| Every drops contains : | |
| Mixed liposomes obtained by sonication and containing : | |
| - N-palmitoylethanolamide | 200 mg |
| - soy lecithin | 100 mg |
| p-hydroxybenzoate | 2 mg |
| purified carrageenin | 40 mg |
| demineralized water | 1.66 g |

| Example 4 Syrup : | |
|---|---|
| 100 g of syrup contain : | |
| Mixed liposomes obtained by sonications and containing : | |
| - N-palmitoylethanolamide | 2 g |
| - soy lecithin | 1 g |
| methyl p-hydroxybenzoate | 0.1 g |
| glycerin | 1.7 g |
| vanilla tincture | 0.15 g |
| orange tincture | 0.6 g |
| saccharose | 19 g |
| demineralized water | q.s. to 100.0 g |

| Example 5 Suppository : | |
|---|---|
| Every suppository contains : | |
| N-palmitoylethanolamide | 30 g |
| soy lecithin | 50 mg |
| triglycerides of fatty acids | q.s. to 1.5 g |

| Example 6 Vials : | |
|---|---|
| Every vial contains : | |
| N-palmitoylethanolamide | 30 mg |
| soy lecithin | 100 mg |
| phosphate buffer pH 7.6 | 2 ml |

| Example 7 Vials : | |
|---|---|
| Every vial contains : | |
| n-palmitoylethanolamide under liposomes form obtained by sonications | 150 mg |
| mannitol | 15 mg |
| bidistilled apyrogen buffered water | q.s. to 1.5 ml |

| Example 8 Lyophilized Tiny Bottle : | |
|---|---|
| Every lyophilized tiny bottle contains : | |
| N-palmitoylethanolamide | 500 mg |
| mannitol | 30 mg |
| glycocoll | 100 mg |
| Every vial contains : | |
| bidistilled apyrogen buffered water | 2.0 ml |

| Example 9 Dermatologic Cream : | |
|---|---|
| 100 g of cream contain : | |
| N-palmitoylethanolamide | 50 mg |
| sorbitan monostearate | 500 mg |
| polyoxyethylen sorbitate | 4.5 g |
| ethanol | 3 g |
| stearic acid | 3 g |
| paraffin oil | 10 g |
| 70 % sorbitol | 6 g |
| p-oxybenzoic acid methyl ester | 0.2 g |
| p-oxybenzoic acid propyl ester | 0.05 g |
| water | q.s. to 100 g |

| Example 10 dietetic integrating component in jelly perles | |
|---|---|
| Every perle contains: | |
| N-palmitoylethanolamide | 30 mg |
| egg lecithin | 90 mg |
| maize oil | 240 mg |

The pharmaceutical compositions containing the compounds according to the present invention may find a valid therapeutic and preventive application in all the pathologies characterized by an injury of the peripheral nerve associated with a neurogenic endoneural oedema substained by mast cells hyperactivation.

In particular the compounds of the present invention can be advantageously administered in the pathologies of the peripheral nervous systems, normally those defined as peripheral somatic and autonomic neuropathies having traumatic, toxic or dysmetabolic origin.

## Claims (Claims for the following Contracting State(s): AT, BE, CH, LI, DE, DK, FR, GB, GR, IE, IT, LU, MC, NL, PT, SE)

1. Use of one or more aminoalcohols N-acylderivatives, having the formula: 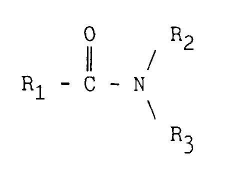 wherein R₂ is an alcoholic residue selected from a C₁-C₂₀ linear or branched hydroxyalkyl, optionally substituted in the alkyl chain with at least one aryl group, and a hydroxyaryl, optionally substituted on the aromatic ring with at least one linear or branched alkyl radical of from 1 to 20 carbon atoms; wherein R₃ is H or 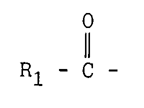 is = R₂; and wherein is an acyl radical of a monocarboxylic acid;
as the active ingredient for the preparation of pharmaceutical compositions for the prevention or the treatment of human and animal pathologies involving a neurogenic endoneural oedema induced by mast cells degranulation as a consequence of a noxa of the peripheral nerve.

2. The use according to claim 1, characterized in that these aminoalcohols residues R₂ and/or R₃ are selected from the group consisting of -CH₂CH₂-OH and

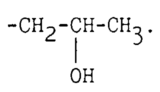

3. The use according to claim 2, characterized in that when R₂ and/or R₃ are 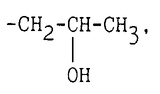 then the N-acylderivative is optically active or raceme.

4. The use according to claim 1, characterized in that 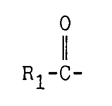 is the acyl radical of a monocarboxylic acid selected from the group consisting of saturated or unsaturated aliphatic acids, having from 2 to 20 carbon atoms, optionally substituted in the aliphatic chain with a amino or a hydroxy group, and monocarboxylic acids containing aromatic and/or heterocyclic groups.

5. The use according to claim 4, characterized in that said aliphatic acids are selected from the group consisting of palmitic, oleic, lauric,stearic, myristic acids and their homologies having an amino or hydroxy substituent, and said acids containing aromatic and/or heterocyclic groups are selected from the group consisting of salicylic, acetylsalicylic, sulphosalicylic, benzoic, trimethoxybenzoic, α-lipoic, retinoic, thenoic, phenylanthranilic and hydroxyphenylacetic acids.

6. The use according to claim 1, characterized in that said pathologies are the peripheral somatic and autonomic neuropathies, having traumatic, toxic or dysmetabolic origin.

7. The use according to claim 1, characterized in that said aminoalcohols N-acylderivatives are to be administered orally in the form of dry powder and are selected from the group consisting of granulates, tablets, dragees, hard and soft jelly perles; or in the liquid form selected from the group consisting of suspensions, emulsions and aqueous solutions, wherein the active ingredients are present in the form of liposomes, optionally mixed with other lipids; or in semisolid form optionally obtained with polysaccharides aqueous suspensions solutions or emulsions wherein the active ingredient is present in the form of liposomes optionally mixed with other biologically acceptable lipids.

8. The use according to claim 1, characterized in that said aminoalcohols N-acylderivatives are to be administered as dietetic integrating components in the form of dragees, tablets or oily perles, both for human beings and for animals.

9. The use according to claim 1, characterized in that said aminoalcohols N-acylderivatives are to be administered topically, rectally and parenterally.

10. The use according to claim 9, characterized in that said aminoalcohols N-acylderivatives are to be administered parenterally in the form of buffered aqueous solutions or emulsions, optionally containing the active ingredient in the form of liposomes optionally mixed with other biologically acceptable lipids, in the form of oily solutions, or in the form of a lyophilized product dispersable in the solvent at the moment of the administration.

11. The use according to claim 9, characterized in that said aminoalcohols N-acylderivatives are to be administered rectally in the form of dispersions in a biologically acceptable phospholipidic excipient.

12. The use according to claim 9, characterized in that said aminoalcohols N-acylderivatives are to be administered topically in the form of buffered solutions, gels or medicated plasters.

13. The use according to claim 1, characterized in that said aminoalcohols N-acylderivatives are to be administered in doses ranging from 0.1 to 50 mg/kg.

14. The use according to claim 13, characterized in that said doses range from 1 to 20 mg/kg.

15. The use according to claim 13, characterized in that said doses are to be administered from once to twice a day, for a period of 3-4 weeks.

16. The use according to claim 8, characterized in that said aminoalcohols N-acylderivatives are to be administered in daily doses ranging from 0.1 to 1 mg/kg.

## Claims (Claims for the following Contracting State(s): ES)

1. Process to obtain a pharmaceutical composition useful for the prevention or the treatment of human and animal pathologies involving a neurogenic endoneural oedema induced by mast cells degranulation as a consequence of a noxa of the peripheral nerve, said process consisting in mixing one or more aminoalcohols N-acylderivatives, having the formula: 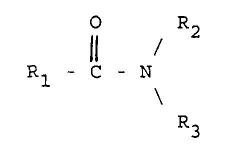 wherein R₂ is an alcoholic residue selected from a C₁-C₂₀ linear or branched hydroxyalkyl optionally substituted in the alkyl chain with at least one aryl group, and a hydroxyaryl, optionally substituted on the aromatic ring with at least one linear or branched alkyl radical of from 1 to 20 carbon atoms, wherein R₃ is H or is = R₂; and wherein 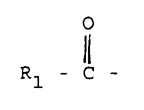 is an acyl radical of a monocarboxylic acid, with one or more pharmaceutically acceptable excipients.

2. The process according to claim 1, characterized in that these aminoalcohols residues R₂ and/or R₃ are selected from the group consisting of -CH₂CH₂-OH and

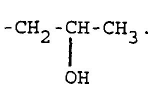

3. The process according to claim 2, characterized in that when R₂ and /or R₃ are 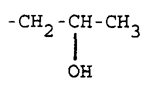 then the N-acylderivative is an optically active or raceme.

4. The process according to claim 1 characterized in that 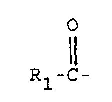 is the acyl radical of a monocarboxylic acid selected from the group consisting of saturated or unsaturated aliphatic acids, having from 2 to 20 carbon atoms, optionally substituted in the aliphatic chain with a amino or a hydroxy group, and monocarboxylic acids containing aromatic and/or heterocyclic groups.

5. The process according to claim 4, characterized in that said aliphatic acids are selected from the group consisting of palmitic, oleic, lauric, stearic, myristic acids and their homologues having an amino or hydroxy substituent, and said acids containing aromatic and/or heterocyclic groups are selected from the group consisting of salicylic, acetylsalicylic, sulphosalicylic, benzoic, trimethoxybenzoic, α-lipoic, retinoic, thenoic, phenylanthranilic, hydroxyphenylacetic acids.

6. The process according to claim 1, characterized in that said pathologies are the peripheral somatic and autonomic neuropathies having traumatic, toxic or dysmetabolic origin.

7. The process according to claim 1, characterized in that said aminoalcohols N-acylderivatives are to be administered orally in the form of dry powder and are selected from the group consisting of granulates, tablets, dragees, hard or soft jelly perles; or in the liquid form selected from the group consisting of suspensions, emulsions and aqueous solutions, wherein the active ingredients are present in the form of liposomes, optionally mixed with other lipids; or in semisolid form optionally obtained with polysaccharides, aqueous suspensions, solutions or emulsions wherein the active ingredient is present in the form of liposomes optionally mixed with other biologically acceptable lipids.

8. The process according to claim 1, characterized in that said aminoalcohols N-acylderivatives are to be administered as dietetic integrating components in the form of dragees, tablets or oily perles, both for human beings and for animals.

9. The process according to claim 1, characterized in that said aminoalcohols N-acylderivatives are to be administered topically, rectally and parenterally.

10. The process according to claim 9, characterized in that said aminoalcohols N-acylderivatives are to be administered parenterally in the form of buffered aqueous solutions or emulsions, optionally containing the active ingredient in the form of liposomes optionally mixed with other biologically acceptable lipids, in the form of oily solutions, or in the form of a lyophilized product dispersable in the solvent at the moment of the administration.

11. The process according to claim 9, characterized in that said aminoalcohols N-acylderivatives are to be administered rectally in the form of dispersions in a biologically acceptable phospholipidic excipient.

12. The process according to claim 9, characterized in that said aminoalcohols N-acylderivatives are to be administered topically in the form of buffered solutions, gels or medicated plasters.

13. The process according to claim 1, characterized in that said aminalcohols N-acylderivatives are to be administered in dosages ranging from 0.1 to 50 mg/kg.

14. The process according to claim 13, charcterized in that said doses range from 1 to 20 mg/kg.

15. The process according to claim 13, characterized in that said doses are to be administered from once to twice a day, for a period of 3-4 weeks.

16. The process according to claim 8, characterized in that said aminoalcohols N-acylderivatives are to be administered in daily doses ranging from 0.1 to 1 mg/kg.

## Patentansprüche (Patentansprüche für folgende(n) Vertragsstaat(en): AT, BE, CH, LI, DE, DK, FR, GB, GR, IE, IT, LU, MC, NL, PT, SE)

1. Verwendung eines oder mehrerer Aminoalkohol-N-acylderivate der Formel: 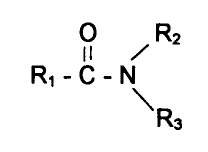 worin bedeuten:
R₂ einen alkoholischen Rest, ausgewählt aus einem linearen oder verzweigten C₁-C₂₀-Hydroxyalkyl, das gegebenenfalls in der Alkylkette durch mindestens eine Arylgruppe substituiert ist, und einem Hydroxyaryl, das gegebenenfalls an dem aromatischen Ring durch mindestens einen linearen oder verzweigten Alkylrest mit 1 bis 20 Kohlenstoffatomen substituiert ist;
R₃ H oder = R₂; und 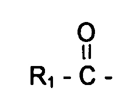
einen Acylrest einer Monocarbonsäure; als Wirkstoff (aktiven Bestandteil) für die Herstellung pharmazeutischer Zusammensetzungen zur Prävention oder Behandlung von Human- und Tier-Pathologien, bei denen ein neuroges Endoneural-Ödem auftritt, das durch eine Mastzellen-Degranulation als Folge einer Schädigung des peripheren Nervs induziert worden ist.

2. Verwendung nach Anspruch 1, dadurch gekennzeichnet, daß diese Aminoalkohol-Reste R₂ und/oder R₃ ausgewählt werden aus der Gruppe, die besteht aus -CH₂CH₂-OH und

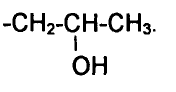

3. Verwendung nach Anspruch 2, dadurch gekennzeichnet, daß dann, wenn R₂ und/oder R₃ für 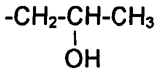 steht (stehen),
das N-Acylderivat optisch aktiv oder racemisch ist.

4. Verwendung nach Anspruch 1, dadurch gekennzeichnet, daß 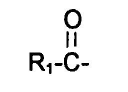 den Acylrest einer Monocarbonsäure darstellt, die ausgewählt wird aus der Gruppe, die besteht aus gesättigten oder ungesättigten aliphatischen Säuren mit 2 bis 20 Kohlenstoffatomen, die gegebenenfalls in der aliphatischen Kette durch eine Amino- oder eine Hydroxygruppe substituiert sind, und Monocarbonsäuren, die aromatische und/oder heterocyclische Gruppen enthalten.

5. Verwendung nach Anspruch 4, dadurch gekennzeichnet, daß die genannten aliphatischen Säuren ausgewählt werden aus der Gruppe, die besteht aus Palmitin-, Öl-, Laurin-, Stearin- und Myristinsäure und ihren Homologen, die einen Amino- oder Hydroxy-Substituenten aufweisen, und daß die genannten Säuren, die aromatische und/oder heterocyclische Gruppen enthalten, ausgewählt werden aus der Gruppe, die besteht aus Salicyl-, Acetylsalicyl-, Sulfosalicyl-, Benzoe-, Trimethoxybenzoe-, α-Lipon-, Retinoe-, Thenoyl-, Phenylanthranil- und Hydroxyphenylessigsäure.

6. Verwendung nach Anspruch 1, dadurch gekennzeichnet, daß die genannten Pathologien periphere somatische und autonome Neuropathien sind, die traumatischen, toxischen oder dysmetabolischen Ursprungs sind.

7. Verwendung nach Anspruch 1, dadurch gekennzeichnet, daß die genannten Aminoalkohol-N-acylderivate oral verabreicht werden sollen in Form eines trockenen Pulvers ausgewählt aus der Gruppe, die besteht aus Granulaten, Tabletten, Dragees, harten und weichen Gelee-Perlen; oder in flüssiger Form, ausgewählt aus der Gruppe, die besteht aus Suspensionen, Emulsionen und wäßrigen Lösungen, wobei die Wirkstoffe (aktiven Bestandteile) in Form von Liposomen vorliegen, die gegebenenfalls mit anderen Lipiden gemischt sind; oder in halbfester Form, die gegebenenfalls mit wäßrigen Polysaccharid-Suspensionen, -Lösungen oder -Emulsionen erhalten wird, in der der Wirkstoff (aktive Bestandteil) in Form von Liposomen vorliegt, die gegebenenfalls mit anderen (weiteren) biologisch akzeptablen Lipiden gemischt sind.

8. Verwendung nach Anspruch 1, dadurch gekennzeichnet, daß die genannten Aminoalkohol-N-acylderivate als diätetische Integrations-Komponenten in Form von Dragees, Tabletten oder öligen Perlen sowohl an Menschen als auch an Tiere verabreicht werden sollen.

9. Verwendung nach Anspruch 1, dadurch gekennzeichnet, daß die genannten Aminoalkohol-N-acylderivate topisch, rektal und parenteral verabreicht werden sollen.

10. Verwendung nach Anspruch 9, dadurch gekennzeichnet, daß die genannten Aminoalkohol-N-acylderivate parenteral verabreicht werden sollen in Form von gepufferten wäßrigen Lösungen oder Emulsionen, die gegebenenfalls den Wirkstoff (aktiven Bestandteil) in Form von Liposomen, gegebenenfalls im Gemisch mit anderen biologisch akzeptablen Lipiden, enthalten, in Form von öligen Lösungen oder in Form eines lyophilisierten Produkts, das im Augenblick der Verabreichung in dem Lösungsmittel dispergierbar ist.

11. Verwendung nach Anspruch 9, dadurch gekennzeichnet, daß die genannten Aminoalkohol-N-acylderivate rektal verabreicht werden sollen in Form von Dispersionen in einem biologisch akzeptablen Phospholipid-Exzipienten.

12. Verwendung nach Anspruch 9, dadurch gekennzeichnet, daß die genannten Aminoalkohol-N-acylderivate in Form von gepufferten Lösungen, Gelen oder medizinischen Pflastern topisch verabreicht werden sollen.

13. Verwendung nach Anspruch 1, dadurch gekennzeichnet, daß die genannten Aminoalkohol-N-acylderivate in Dosen in dem Bereich von 0,1 bis 50 mg/kg verabreicht werden sollen.

14. Verwendung nach Anspruch 13, dadurch gekennzeichnet, daß die genannten Dosen in dem Bereich von 1 bis 20 mg/kg liegen.

15. Verwendung nach Anspruch 13, dadurch gekennzeichnet, daß die genannten Dosen ein- bis zweimal pro Tag für eine Zeitspanne von 3 bis 4 Wochen verabreicht werden sollen.

16. Verwendung nach Anspruch 8, dadurch gekennzeichnet, daß die genannten Aminoalkohol-N-acylderivate in täglichen Dosen in dem Bereich von 0,1 bis 1 mg/kg verabreicht werden sollen.

## Patentansprüche (Patentansprüche für folgende(n) Vertragsstaat(en): ES)

1. Verfahren zur Erhaltung einer pharmazeutischen Zusammensetzung geeignet zur Prävention oder Behandlung von Human- und Tierpathologien, bei denen ein neuroges Endoneural-Ödem auftritt, das durch eine Mastzellen-Degranulation als Folge einer Schädigung des peripheren Nervs induziert worden ist, dieses Verfahren bestehend aus der Mischung eines oder mehrerer Aminoalkohol-N-acylderivate der Formel: 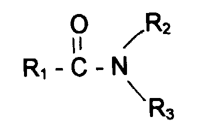 worin bedeuten:
R₂ einen alkoholischen Rest, ausgewählt aus einem linearen oder verzweigten C₁-C₂₀-Hydroxyalkyl, das gegebenenfalls in der Alkylkette durch mindestens eine Arylgruppe substituiert ist, und einem Hydroxyaryl, das gegebenenfalls an dem aromatischen Ring durch mindestens einen linearen oder verzweigten Alkylrest mit 1 bis 20 Kohlenstoffatomen substituiert ist;
R₃ H oder = R₂; und 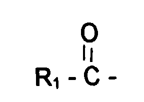
einen Acylrest einer Monocarbonsäure, mit einem oder mehreren pharmazeutisch akzeptablen Exzipienten.

2. Verfahren nach Anspruch 1, dadurch gekennzeichnet, daß diese Aminoalkohol-Reste R₂ und/oder R₃ ausgewählt werden aus der Gruppe, die besteht aus -CH₂CH₂-OH und

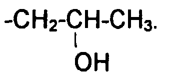

3. Verfahren nach Anspruch 2, dadurch gekennzeichnet, daß dann, wenn R₂ und/oder R₃ für 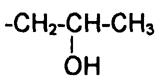 steht (stehen),
das N-Acylderivat optisch aktiv oder racemisch ist.

4. Verfahren nach Anspruch 1, dadurch gekennzeichnet, daß 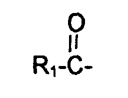 den Acylrest einer Monocarbonsäure darstellt, die ausgewählt wird aus der Gruppe, die besteht aus gesättigten oder ungesättigten aliphatischen Säuren mit 2 bis 20 Kohlenstoffatomen, die gegebenenfalls in der aliphatischen Kette durch eine Amino- oder eine Hydroxygruppe substituiert sind, und Monocarbonsäuren, die aromatische und/oder heterocyclische Gruppen enthalten.

5. Verfahren nach Anspruch 4, dadurch gekennzeichnet, daß die genannten aliphatischen Säuren ausgewählt werden aus der Gruppe, die besteht aus Palmitin-, Öl-, Laurin-, Stearin- und Myristinsäure und ihren Homologen, die einen Amino- oder Hydroxy-Substituenten aufweisen, und daß die genannten Säuren, die aromatische und/oder heterocyclische Gruppen enthalten, ausgewählt werden aus der Gruppe, die besteht aus Salicyl-, Acetylsalicyl-, Sulfosalicyl-, Benzoe-, Trimethoxybenzoe-, α-Lipon-, Retinoe-, Thenoyl-, Phenylanthranil- und Hydroxyphenylessigsäure.

6. Verfahren nach Anspruch 1, dadurch gekennzeichnet, daß die genannten Pathologien periphere somatische und autonome Neuropathien sind, die traumatischen, toxischen oder dysmetabolischen Ursprungs sind.

7. Verfahren nach Anspruch 1, dadurch gekennzeichnet, daß die genannten Aminoalkohol-N-acylderivate oral verabreicht werden sollen in Form eines trockenen Pulvers ausgewählt aus der Gruppe, die besteht aus Granulaten, Tabletten, Dragees, harten und weichen Gelee-Perlen; oder in flüssiger Form, ausgewählt aus der Gruppe, die besteht aus Suspensionen, Emulsionen und wäßrigen Lösungen, wobei die Wirkstoffe (aktiven Bestandteile) in Form von Liposomen vorliegen, die gegebenenfalls mit anderen Lipiden gemischt sind; oder in halbfester Form, die gegebenenfalls mit wäßrigen Polysaccharid-Suspensionen, -Lösungen oder -Emulsionen erhalten wird, in der der Wirkstoff (aktive Bestandteil) in Form von Liposomen vorliegt, die gegebenenfalls mit anderen (weiteren) biologisch akzeptablen Lipiden gemischt sind.

8. Verfahren nach Anspruch 1, dadurch gekennzeichnet, daß die genannten Aminoalkohol-N-acylderivate als diätetische Integrations-Komponenten in Form von Dragees, Tabletten oder öligen Perlen sowohl an Menschen als auch an Tiere verabreicht werden sollen.

9. Verfahren nach Anspruch 1, dadurch gekennzeichnet, daß die genannten Aminoalkohol-N-acylderivate topisch, rektal und parenteral verabreicht werden sollen.

10. Verfahren nach Anspruch 9, dadurch gekennzeichnet, daß die genannten Aminoalkohol-N-acylderivate parenteral verabreicht werden sollen in Form von gepufferten wäßrigen Lösungen oder Emulsionen, die gegebenenfalls den Wirkstoff (aktiven Bestandteil) in Form von Liposomen, gegebenenfalls im Gemisch mit anderen biologisch akzeptablen Lipiden, enthalten, in Form von öligen Lösungen oder in Form eines lyophilisierten Produkts, das im Augenblick der Verabreichung in dem Lösungsmittel dispergierbar ist.

11. Verfahren nach Anspruch 9, dadurch gekennzeichnet, daß die genannten Aminoalkohol-N-acylderivate rektal verabreicht werden sollen in Form von Dispersionen in einem biologisch akzeptablen Phospholipid-Exzipienten.

12. Verfahren nach Anspruch 9, dadurch gekennzeichnet, daß die genannten Aminoalkohol-N-acylderivate in Form von gepufferten Lösungen, Gelen oder medizinischen Pflastern topisch verabreicht werden sollen.

13. Verfahren nach Anspruch 1, dadurch gekennzeichnet, daß die genannten Aminoalkohol-N-acylderivate in Dosen in dem Bereich von 0,1 bis 50 mg/kg verabreicht werden sollen.

14. Verfahren nach Anspruch 13, dadurch gekennzeichnet, daß die genannten Dosen in dem Bereich von 1 bis 20 mg/kg liegen.

15. Verfahren nach Anspruch 13, dadurch gekennzeichnet, daß die genannten Dosen ein- bis zweimal pro Tag für eine Zeitspanne von 3 bis 4 Wochen verabreicht werden sollen.

16. Verfahren nach Anspruch 8, dadurch gekennzeichnet, daß die genannten Aminoalkohol-N-acylderivate in täglichen Dosen in dem Bereich von 0,1 bis 1 mg/kg verabreicht werden sollen.

## Revendications (Revendications pour l'(les) Etat(s) contractant(s) suivant(s): AT, BE, CH, LI, DE, DK, FR, GB, GR, IE, IT, LU, MC, NL, PT, SE)

1. Utilisation d'un ou plusieurs dérivés N-acylés d'aminoalcools de formule 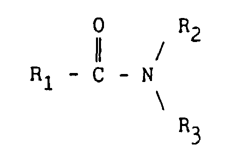 dans laquelle R₂ est un résidu alcoolique choisi parmi un groupement hydroxyalkyle en C₁-C₂₀ linéaire ou ramifié, éventuellement substitué dans la chaîne alkyle par au moins un groupement aryle, et un groupement hydroxyaryle éventuellement substitué sur le cycle aromatique par au moins un radical alkyle linéaire ou ramifié ayant 1 à 20 atomes de carbone ; dans laquelle R₃ est H ou =R₂; et dans laquelle 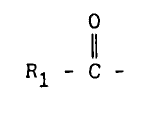 est un radical acyle d'un acide monocarboxylique ;
en tant qu'ingrédient actif pour la préparation de compositions pharmaceutiques pour la prévention ou le traitement de pathologies animales et humaines impliquant un oedème endoneural neurogène induit par une dégranulation de mastocytes en conséquence d'un noxa des nerfs périphériques.

2. Utilisation selon la revendication 1, caractérisée en ce que les résidus aminoalcooliques R₂ et/ou R₃ sont choisis dans le groupe formé par -CH₂-CH₂-OH et

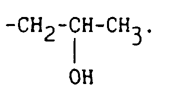

3. Utilisation selon la revendication 2, caractérisée en ce que lorsque R₂ et/ou R₃ sont 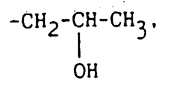 le dérivé N-acylé est optiquement actif ou est un composé racémique.

4. Utilisation selon la revendication 1 caractérisée en ce que 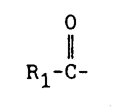 est le radical acyle d'un acide monocarboxylique choisi dans le groupe constitué des acides aliphatiques saturés ou insaturés, ayant de 2 à 20 atomes de carbone, éventuellement substitués dans la chaîne aliphatique par un groupement amino ou hydroxy, et des acides monocarboxyliques contenant des groupements aromatiques et/ou hétérocycliques.

5. Utilisation selon la revendication 4, caractérisée en ce que lesdits acides aliphatiques sont choisis dans le groupe constitué des acides palmitique, oléique, laurique, stéarique et myristique et de leurs homologues ayant un substituant amino ou hydroxy, et lesdits acides contenant des groupements aromatiques et/ou hétérocycliques sont choisis dans le groupe constitué par les acides salicylique, acétylsalicyclique, sulfosalicylique, benzoïque, triméthoxybenzoïque, α-lipoïque, rétinoïque, thénoïque, phénylanthranylique et hydroxyphénylacétique.

6. Utilisation selon la revendication 1, caractérisée en ce que lesdites pathologies sont les neuropathies périphériques somatiques et autonomes, ayant une origine traumatique, toxique ou dysmétabolique.

7. Utilisation selon la revendication 1, caractérisée en ce que lesdits dérivés N-acylés d'aminoalcools sont destinés à être administrés par voie orale sous forme de poudre sèche et sont choisis dans le groupe constitué par les granulés, les comprimés, les dragées, les perles de gelée dures et molles ; ou sous forme liquide et sont choisis dans le groupe constitué par les suspensions, les émulsions et les solutions aqueuses, où les ingrédients actifs sont présents sous forme de liposomes, éventuellement mélangés avec d'autres lipides ; ou sous forme semi-solide éventuellement obtenue avec des suspensions, des solutions ou des émulsions aqueuses de polysaccharides dans lesquelles l'ingrédient actif est présent sous forme de liposomes éventuellement mélangés avec d'autres lipides acceptables biologiquement.

8. Utilisation selon la revendication 1, caractérisée en ce que lesdits dérivés N-acylés d'aminoalcools sont destinés à être administrés en tant que composants d'intégration diététique sous forme de dragées, de comprimés ou de perles huileuses, à la fois pour les hommes et pour les animaux.

9. Utilisation selon la revendication 1, caractérisée en ce que lesdits dérivés N-acylés d'aminoalcools sont destinés à être administrés en topique, par voie rectale et parentérale.

10. Utilisation selon la revendication 9, caractérisée en ce que lesdits dérivés N-acylés d'aminoalcools sont destinés à être administrés par voie parentérale sous forme d'émulsions ou de solutions aqueuses tamponnées, contenant éventuellement l'ingrédient actif sous forme de liposomes éventuellement mélangés avec d'autres lipides acceptables biologiquement, sous forme de solutions huileuses, ou sous forme d'un produit lyophilisé dispersable dans le solvant au moment de l'administration.

11. Utilisation selon la revendication 9, caractérisée en ce que lesdits dérivés N-acylés d'aminoalcools sont destinés à être administrés par voie rectale sous forme de dispersions dans un excipient phospholipidique acceptable biologiquement.

12. Utilisation selon la revendication 9, caractérisée en ce que lesdits dérivés N-acylés d'aminoalcools sont destinés à être administrés par voie topique sous forme de solutions tamponnées, de gels ou de plâtres médicaux.

13. Utilisation selon la revendication 1, caractérisée en ce que lesdits dérivés N-acylés d'aminoalcools sont destinés à être administrés à des doses allant de 0,1 à 50 mg/kg.

14. Utilisation selon la revendication 13, caractérisée en ce que lesdites doses vont de 1 à 20 mg/kg.

15. Utilisation selon la revendication 13, caractérisee en ce que lesdites doses sont destinées à être administrées de une à deux fois par jour, pendant 3 à 4 semaines.

16. Utilisation selon la revendication 8, caractérisée en ce que lesdits dérivés N-acylés d'aminoalcools sont destinés à être administrés en doses journalières allant de 0,1 à 1 mg/kg.

## Revendications (Revendications pour l'(les) Etat(s) contractant(s) suivant(s): ES)

1. Procédé pour préparer une composition pharmaceutique pour la prévention ou le traitement de pathologies animales et humaines impliquant un oedème endoneural neurogène induit par une dégranulation de mastocytes en conséquence d'un noxa des nerfs périphériques, ce procédé étant constitué par le mixage d'un ou plusieurs dérivés N-acylés d'aminoalcools de formule 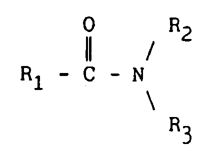 dans laquelle R₂ est un résidu alcoolique choisi parmi un groupement hydroxyalkyle en C₁-C₂₀ linéaire ou ramifié, éventuellement substitué dans la chaîne alkyle par au moins un groupement aryle, et un groupement hydroxyaryle éventuellement substitué sur le cycle aromatique par au moins un radical alkyle linéaire ou ramifié ayant 1 à 20 atomes de carbone ; dans laquelle R₃ est H ou =R₂ ; et dans laquelle 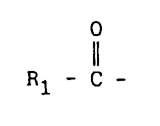 est un radical acyle d'un acide monocarboxylique avec un ou plusieurs excipients acceptables pharmaceutiquement.

2. Procédé selon la revendication 1, caractérisée en ce que les résidus aminoalcooliques R₂ et/ou R₃ sont choisis dans le groupe formé par -CH₂-CH₂-OH et

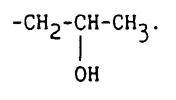

3. Procédé selon la revendication 2, caractérisée en ce que lorsque R₂ et/ou R₃ sont 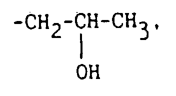 le dérivé N-acylé est optiquement actif ou est un composé racémique.

4. Procédé selon la revendication 1, caractérisée en ce que 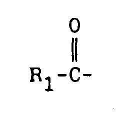 est le radical acyle d'un acide monocarboxylique choisi dans le groupe constitué des acides aliphatiques saturés ou insaturés, ayant de 2 à 20 atomes de carbone, éventuellement substitués dans la chaîne aliphatique par un groupement amino ou hydroxy, et des acides monocarboxyliques contenant des groupements aromatiques et/ou hétérocycliques.

5. Procédé selon la revendication 4, caractérisée en ce que lesdits acides aliphatiques sont choisis dans le groupe constitué des acides palmitique, oléique, laurique, stéarique et myristique et de leurs homologues ayant un substituant amino ou hydroxy, et lesdits acides contenant des groupements aromatiques et/ou hétérocycliques sont choisis dans le groupe constitué par les acides salicylique, acétylsalicylique, sulfosalicylique, benzoïque, triméthoxybenzoïque, α-lipoïque, rétinoïque, thénoïque, phénylanthranylique et hydroxyphénylacétique.

6. Procédé selon la revendication 1, caractérisée en ce que lesdites pathologies sont les neuropathies périphériques somatiques et autonomes, ayant une origine traumatique, toxique ou dysmétabolique.

7. Procédé selon la revendication 1, caractérisée en ce que lesdits dérivés N-acylés d'aminoalcools sont destinés à être administrés par voie orale sous forme de poudre sèche et sont choisis dans le groupe constitué par les granulés, les comprimés, les dragées, les perles de gelée dures et molles ; ou sous forme liquide et sont choisis dans le groupe constitué par les suspensions, les émulsions et les solutions aqueuses, où les ingrédients actifs sont présents sous forme de liposomes, éventuellement mélangés avec d'autres lipides ; ou sous forme semi-solide éventuellement obtenue avec des suspensions, des solutions ou des émulsions aqueuses de polysaccharides dans lesquelles l'ingrédient actif est présent sous forme de liposomes éventuellement mélangés avec d'autres lipides acceptables biologiquement.

8. Procédé selon la revendication 1, caractérisée en ce que lesdits dérivés N-acylés d'aminoalcools sont destinés à être administrés en tant que composants d'intégration diététique sous forme de dragées, de comprimés ou de perles huileuses, à la fois pour les hommes et pour les animaux.

9. Procédé selon la revendication 1, caractérisée en ce que lesdits dérivés N-acylés d'aminoalcools sont destinés à être administrés en topique, par voie rectale et parentérale.

10. Procédé selon la revendication 9, caractérisée en ce que lesdits dérivés N-acylés d'aminoalcools sont destinés à être administrés par voie parentérale sous forme d'émulsions ou de solutions aqueuses tamponnées, contenant éventuellement l'ingrédient actif sous forme de liposomes éventuellement mélangés avec d'autres lipides acceptables biologiquement, sous forme de solutions huileuses, ou sous forme d'un produit lyophilisé dispersable dans le solvant au moment de l'administration.

11. Procédé selon la revendication 9, caractérisée en ce que lesdits dérivés N-acylés d'aminoalcools sont destinés à être administrés par voie rectale sous forme de dispersions dans un excipient phospholipidique acceptable biologiquement.

12. Procédé selon la revendication 9, caractérisée en ce que lesdits dérivés N-acylés d'aminoalcools sont destinés à être administrés par voie topique sous forme de solutions tamponnées, de gels ou de plâtres médicaux.

13. Procédé selon la revendication 1, caractérisée en ce que lesdits dérivés N-acylés d'aminoalcools sont destinés à être administrés à des doses allant de 0,1 à 50 mg/kg.

14. Procédé selon la revendication 13, caractérisée en ce que lesdites doses vont de 1 à 20 mg/kg.

15. Procédé selon la revendication 13, caractérisée en ce que lesdites doses sont destinées à être administrées de une à deux fois par jour, pendant 3 à 4 semaines.

16. Procédé selon la revendication 8, caractérisée en ce que lesdits dérivés N-acylés d'aminoalcools sont destinés à être administrés en doses journalières allant de 0,1 à 1 mg/kg.
